# EUROPÄISCHE PATENTSCHRIFT

(11) **EP 2 399 621 B1**
(45) Veröffentlichungstag und Bekanntmachung des Hinweises auf die Patenterteilung: **20.07.2016**
(21) Anmeldenummer: 10167402.6
(22) Anmeldetag: 25.06.2010
(51) Int. Cl.: A61M 5/14, A61M 5/38

(54) **Tropfkammer mit Lüftungsventil**
Drip chamber with ventilation valve
Chambre de goutte-à-goutte dotée d'une soupape d'aération

(43) Veröffentlichungstag der Anmeldung: 28.12.2011
(73) Patentinhaber: Codan Holding GmbH, 23738 Lensahn (DE)
(72) Erfinder: Krüger, Peter, 23738 Damlos (DE); Ahrens, Helge, 24223, Schwentinental (DE)
(74) Vertreter: Raffay & Fleck

(56) Entgegenhaltungen:
- WO-A1-01/41844
- DE-A1- 19 748 497
- US-A- 5 242 424
- US-A1- 2009 259 199
- US-B1- 6 336 916

## Beschreibung

Die vorliegende Erfindung betrifft eine Tropfkammer für ein Infusionsbesteck zur Abgabe einer Infusions- und oder Medikamentenflüssigkeit an einen Patienten nach dem Oberbegriff des Anspruchs 1. Eine derartige Tropfkammer bzw. ein derartiger Diffusor wird in den US 2009/259 199 A1 und US 5 242 424 A beschrieben.

Tropfkammern und Infusionsbestecke mit Tropfkammern sind aus dem Stand der Technik seit langem bekannt. Eine Tropfkammer dient dabei zur Vermeidung der Überführung von Gasblasen in die Blutbahn des Patienten. Dazu ist es erforderlich, dass die Tropfkammer in ihrem Tropfkammervolumen einen entsprechenden Flüssigkeitsstand aufweist. Ein solcher ist bei Beginn der Infusion herzustellen und auch bei Wechsel einer Infusion oder Arbeiten am Infusionsbesteck vor Wiederaufnahme der Infusion wiederherzustellen, um schwerwiegende Schäden beim Patienten zu vermeiden.

Bei den bekannten Tropfkammern für Infusionsbestecke sind zwei Arten solcher zu unterscheiden. Zum einen sind Tropfkammern für einfache Anwendungen ohne Nebenlinien teilweise so ausgebildet, dass sie direkt in ein Infusionsbehältnis eingestochen werden können und dazu direkt mit der Tropfkammer verbunden einen Einstechdorn aufweisen. In solchen Fällen ist eine direkte Rückbelüftung durch einen zweiten Einstechdorn bekannt.

Auf der anderen Seite gibt es im Stand der Technik Tropfkammern, die über einen Schlauch, gegebenenfalls mit dazwischen liegenden Konnektoren letztendlich durch einen Einstechdorn mit einem Infusionsbehältnis verbunden werden. Bei solchen Infusionsbestecken ist die Tropfkammer in der Regel flexibel ausgebildet, sodass nach Schließen des Patientenanschlusses durch Zusammendrücken der Tropfkammer aus der Tropfkammer in die Infusion zurückgedrückt werden kann und dass beim Loslassen freiwerdende Tropfkammervolumen zumindest teilweise mit Infusionsflüssigkeit gefüllt wird. Dies bedingt die Gefahr, dass bei nicht verschlossenem Patientenanschluss Luft oder Infusion mit Druck in die Blutbahn des Patienten gebracht wird und ist ansonsten auch vergleichsweise umständlich, da insbesondere bei längeren Zuflussschläuchen nur schwer Luft aus der Tropfkammer in das Infusionsbehältnis zurückgedrückt werden kann und beim Loslassen der Tropfkammer nur sehr wenig Infusionsflüssigkeit in die Tropfkammer gesaugt wird, sondern vielmehr zunächst die noch im Zuführschlauch befindliche Luft wieder zurück in die Tropfkammer gelangt. Aufgabe der vorliegenden Erfindung ist es somit, die eingangs genannte Tropfkammer so zu verbessern, dass aus ihr sicher, schnell und einfach Luft und/oder Abgase entfernt und aufgefangen werden können und das Flüssigkeitsniveau in der Tropfkammer durch Nachfüllen von Infusionsflüssigkeit auf das normale bzw. gewünschte Niveau gebracht werden kann, und auch das anfängliche Füllen der Tropfkammer und des Linienschlauches einfach, sicher und schnell durchführbar ist.

Gelöst wird diese Aufgabe durch eine Tropfkammer gemäß Anspruch 1. Die abhängigen Unteransprüche 2 bis 12 geben vorteilhafte Weiterbildungen an.

Erfindungsgemäß ist ein flexibler Ballon zur Aufnahme der durch die mindestens eine Durchlassöffnung austretenden Abgase und/oder Aerosole vorgesehen. Bei einem solchen Entlüftungsgas handelt es sich beispielsweise um Gase, die aus der Infusionslösung ausdampfen oder um Aerosole der Infusionslösung, die jedoch zumindest zum überwiegenden Teil schon durch ein 0,2µ hydrophoben Filter zurück gehalten werden können.

Ein solcher Ballon ist somit so zu befestigen, dass die üblicherweise austretenden Entlüftungsgase sicher in diesem Ballon aufgefangen werden. Dazu wird er mit Vorteil umschließend und gasdicht mit dem oberen Ende der Tropfkammer verbunden. Dabei können die Bedienelemente auch innerhalb des flexiblen Ballons angeordnet sein und trotzdem von außen bedienbar sein. Dies ermöglicht eine besonders einfache Ausführung des Lüftungsventils bei gleichzeitiger Sicherstellung der vollständigen Auffangung der Entlüftungsgase. Wird bei einer solchen Anordnung der Ballon prall, so kann eine weitere Bedienung unter Umständen nicht möglich sein. In einem solchen Fall verschließt sich das Lüftungsventil automatisch.

Zur Verwendung eines solchen flexiblen Ballons wird am Zuführschlauchanschluss zunächst ein Rohr oder ein Schlauch mit einer Muffe befestigt, wobei an der Muffe der Anschluss für einen Zuführschlauch vorgesehen ist. In einer solchen Anordnung ist der Ballon an der Muffe fixiert, sodass der Ballon dann an seinem unteren Ende beispielsweise am oberen Ende der Tropfkammer fixiert und an einem anderen Punkt an der Muffe befestigt ist.

Dieses verhindert, dass der Ballon in der Gegend herumwandert und bietet ihm vielmehr eine vordefinierte Lage. Dieses verhindert Störungen durch einen lose angebrachten und herumwackelnden Ballon. Darüber hinaus wird dadurch auch die Bruchsicherheit des Ballons aufgrund der Einschränkung seiner Bewegung verbessert.

Die Tropfkammer sowie ein Infusionsbesteck zur Abgabe einer Infusion und/oder Medikamenten-Flüssigkeit an einen Patienten weist einen an einem oberen Ende der Tropfkammer angeordneten Zuführschlauchanschluss zum Anschluss eines Zuführschlauches und einen an einem unteren Ende der Tropfkammer angeordneten Auslassschlauchanschluss zum Anschluss eines Auslassschlauches auf. Der Zuführschlauchanschluss ist in der Regel, gegebenenfalls über dazwischen liegende Konnektoren, mit einem Einstechdorn zum Einstechen in ein Infusionsbehältnis versehen. Der Auslassschlauchanschluss weist in der Regel am Ende eine Möglichkeit auf, ihn mit einer Infusionskanüle zu verbinden. Dazu wird in der Regel ein Patientenanschluss vorgesehen. Dazwischen liegend sind meist weitere Elemente, wie Hähne oder Durchflussregler angeordnet.

Die Tropfkammer zeichnet sich dadurch aus, dass am oberen Ende der Tropfkammer mindestens eine Durchlassöffnung ausgebildet ist. Eine solche Durchlassöffnung ermöglicht einen Gasstrom aus dem innerhalb der Tropfkammer befindlichen Tropfkammervolumen nach außen. Des Weiteren ist ein Lüftungsventil an dem oberen Ende der Tropfkammer zur Be- und/oder Entgasung derselben vorgesehen. Das Lüftungsventil weist ein elastisches Verschlusselement zum automatischen Verschließen der mindestens einen Durchlassöffnung auf. Dadurch ist die Durchlassöffnung im Ruhezustand verschlossen und es besteht im Ruhezustand gerade kein Gasdurchlass zwischen Tropfkammervolumen und Ballon.

Darüber hinaus weist das Lüftungsventil beispielsweise Bedienflügel zum manuellen elastischen Verformen des Verschlusselementes unter zumindest teilweiser Freigabe der mindestens einen Durchlassöffnung auf. Durch die Bedienelemente kann das Verschlusselement derart verformt werden, dass die Durchlassöffnung zumindest teilweise freigegeben wird. Dadurch kann durch das Bedienen der Bedienflügel und die dadurch bewirkte Verformung des Verschlusselementes eine Öffnung der Durchlassöffnung erreicht werden. Genau gesprochen ist die Durchlassöffnung an sich immer eine Durchlassöffnung. Sie wird vielmehr durch das Verschlusselement blockiert und durch das Bedienen der Bedienflügel und die elastische Verformung des Verschlusselementes freigegeben. Das Blockieren muss dabei nicht durch Abschließen der Durchlassöffnungen direkt am Ende der Durchlassöffnungen geschehen. Es kommt lediglich darauf an, dass die Verbindung der Durchlassöffnungen zum Ballon durch das Verschlusselement unterbunden wird.

Vorteilhafterweise wird im Lüftungsventil ein insbesondere umlaufender Lüftungskanal ausgebildet, der mit den Durchlassöffnungen verbunden ist, so dass ein Verbinden der Durchlassöffnungen mit dem Ballon beim Öffnen, vermittelt über den Lüftungskanal, unabhängig von der Geometrie der Verformung ermöglicht wird. Bei einer solchen Ausbildung weist das Lüftungsventil einen auch im Ruhezustand ausgebildeten und mit den Durchlassöffnungen verbundenen Lüftungskanal auf. Er ist vorteilhafterweise umlaufend ausgebildet, so dass auch bei Verdrehen des Lüftungsventils eine Verbindung zwischen Durchlassöffnungen und Lüftungskanal sichergestellt ist. Dieser Lüftungskanal ist im Ruhezustand durch das Verschlusselement zur Umgebung abgedichtet. Dadurch sind die Durchlassöffnungen verschlossen, ihre Verbindung zum Ballon unterbrochen. Durch die elastische Verformung des Verschlusselementes beim Öffnen wird die Abdichtung durchbrochen und der der Lüftungskanal mit dem Ballon verbunden.

Durch die Wahl von zwei Bedienelementen kann verhindert werden, dass bei der Bedienung ein Festhalten der üblicherweise frei an Schläuchen hängenden Tropfkammer erforderlich ist oder die Tropfkammer sich unter der auf nur ein Bedienelement ausgeübten Kraft wegbewegt und damit das Bedienen erschwert. Durch eine erfindungsgemäße und technisch einfach zu realisierende Anordnung am oberen Ende der Tropfkammer kann eine im Ruhezustand verschlossene und sich automatisch selbst wieder verschließende Tropfkammer ausgebildet werden, die jedoch einfach zu be- und entlüften ist. Die Wahl von Bedienflügeln ermöglicht eine besonders einfache Bedienung. Das automatische Verschließen ist von Vorteil, da dadurch die Patientensicherheit gesteigert werden kann.

Wird eine solche Tropfkammer zum ersten Mal verwendet und mit einem Infusionsbehältnis verbunden, so kann das Lüftungsventil durch Bedienen der Bedienflügel offengehalten werden und während dieses Offenhaltens kann dann Infusionsflüssigkeit in die Tropfkammer und in den Auslassschlauch fließen. Ist der Auslassschlauch gefüllt und ein gewünschtes Flüssigkeitsniveau in der Tropfkammer erreicht, kann durch einfaches Loslassen der Bedienflügel ein Verschließen des Lüftungsventils erreicht und damit das Infusionsbesteck bzw. die Tropfkammer dem benutzungsfertigen Zustand überführt werden.

Sinkt das Flüssigkeitsniveau in dem Tropfkammervolumen unter ein gewünschtes Maß, kann durch einfaches Bedienen der Bedienflügel ein Entlüften und gleichzeitiges Nachlaufen von Infusionsflüssigkeit erzielt werden. Jedoch ist es auch umgekehrt vorstellbar, dass durch das Lüftungsventil Luft in die Tropfkammer gebracht wird.

Vorteilhafterweise ist das Lüftungsventil gemäß Anspruch 2 so ausgebildet, dass die Bedienflügel mit zwei Fingern unter der manuellen elastischen Verformung zur zumindest teilweisen Freigabe der mindestens einen Durchlassöffnung aufeinander zu bewegbar sind. Bei einer solchen Ausführung können die Bedienflügel mit zwei Fingern einfach bedient werden und dadurch eine zumindest teilweise Freigabe der Durchlassöffnung erreicht werden.

Vorteilhafterweise sind mindestens zwei, insbesondere vier, Durchlassöffnungen gemäß Anspruch 3 vorgesehen. Vorteilhafterweise sind die Durchlassöffnungen auf einem Kreis um den Zuführschlauchanschluss, der in der Regel ebenfalls kreisförmig ausgebildet sein wird, angeordnet. Dies ermöglicht, insbesondere bei Wahl eines ringförmigen elastischen Verschlusselementes, insbesondere gemäß Anspruch 4, ein besonders leichtes Öffnen des Lüftungsventils und ein besonders zuverlässiges Verschließen desselben. Dabei sind vorteilhafterweise die Bedienelemente und das Lüftungsventil im Ruhezustand so angeordnet, dass die Bedienelemente auf jeweils einer Linie ausgehend von der Mitte des Zuführschlauchanschlusses durch eine der Durchlassöffnungen liegen.

Dies ermöglicht eine besonders einfache Ausbildung des Verschlusselementes und ein besonders zuverlässiges Öffnen und Schließen bzw. Freigeben und Blockieren der Durchlassöffnungen.

Darüber hinaus weist sie den Vorteil auf, dass auch bei Verstopfen einer Durchlassöffnung eine Belüftung möglich ist.

Bevorzugt ist das Verschlusselement ringförmig gemäß Anspruch 4 ausgebildet und konzentrisch um den Zuführanschluss angeordnet. Die symmetrische Ausbildung ermöglicht ein besonders einfache Handhabung und Herstellung.

Mit Vorteil sind die Bedienflügel gemäß Anspruch 5 vom Verschlusselement ausgehend radial nach außen erstreckend angeordnet. Dabei schließen sie im Ruhezustand vorteilhafterweise einen Winkel von mindestens 40° und weniger als 180°, insbesondere von 120° ein. Eine solche Anordnung der Bedienflügel ermöglicht eine besonders einfaches und intuitives bedienen der Bedienflügel mit zwei Fingern.

Eine radial nach außen gerichtete Anordnung der Bedienflügel bedeutet jedoch nicht, dass es sich bei den Bedienflügeln um dünne und gerade ausgebildete Elemente handeln muss. Vielmehr können diese auch abgerundet und schließlich in das ringförmige Verschlusselement übergehend ausgebildet sein. Es kommt lediglich darauf an, dass eine durch ihre Längserstreckung gedachte Linie dieses Winkelmaß einschließt.

Mit Vorteil weist das Lüftungsventil gemäß Anspruch 6 einen ringförmigen Befestigungsschnitt zum formschlüssigen Eingriff in eine am oberen Ende der Tropfkammer vorgesehene ringförmige und zum Zuführanschluss konzentrische Vertiefung auf. Dabei kann der Befestigungsschnitt durch einen Teil des Verschlusselements gebildet werden.

Eine solche Ausgestaltung eines Abschnitts des Lüftungsventils bietet den Vorteil, das Lüftungsventil auf einfache Weise mit dem oberen Ende der Tropfkammer verbinden zu können.

Darüber hinaus ermöglicht eine solche ringförmige Ausbildung eines Befestigungsabschnittes, der insbesondere elastisch ausgebildet ist, eine besonders zuverlässige Funktion des Lüftungsventils, da er beim Öffnen des Lüftungsventils mitverformt wird und somit ebenfalls zur Rückstellkraft beiträgt.

Somit kann der Befestigungsabschnitt auch als Verschluss- und/oder Dichtungsabschnitt wirken.

Mit Vorteil ist gemäß Anspruch 7 ein Sicherungselement zur Sicherung des Verschlusselementes vorgesehen und oberhalb und direkt angrenzend an das Verschlusselement angeordnet. In einem solchen Fall weist das Verschlusselement an einem oberen an das Sicherungselement angrenzenden Ende mindestens eine Gasaussparung auf. Durch eine solche Gasaussparung tritt die aus den Durchlassöffnungen austretende und durch das Lüftungsventil hindurch geführte Luft bzw. das entsprechende Gas aus der Anordnung aus. Es ist ebenfalls möglich, solche Gasauslassaussparungen an anderen Stellen vorzusehen. Doch ist die hier beschriebene Form eine besonders einfach auszubildende Ausführung.

Gemäß Anspruch 8 ist vorteilhafterweise eine Filtermembran zwischen dem Tropfkammervolumen und der mindestens einen Durchlassöffnung angeordnet. Eine solche Anordnung verhindert insbesondere dann, wenn aus irgendeinem Grund die Tropfkammer umgedreht oder gekippt werden sollte, ein Austreten von Flüssigkeiten durch das Lüftungsventil.

Mit Vorteil wird als Filtermembran eine 0,2 µ hydrophobe Filtermembran mit vorteilhafterweise 50 bis 120 mm², insbesondere 85 m², wirksamer Filtrationsfläche vorgesehen. Soll eine solche Filtrationsfläche wirksam vorgesehen werden, muss zwischen der Filtermembran und den in der Regel relativ klein ausgebildeten Durchlassöffnungen ein Volumen vorgesehen sein, dass eine entsprechende Kontaktfläche zur Filtermembran aufweist.

Mit Vorteil wird gemäß Anspruch 9 zwischen Tropfkammervolumen und Auslassanschluss ein Auslassfilter, vorzugsweise ein Filtergewebe mit einer Maschenweite von 5 bis 15µ, insbesondere von 15µ, vorgesehen. Dazu wird vorteilhafterweise ein PA (Polyamid)-Filtergewebe verwendet. Alternativ kann eine ein hydrophiler PES (Polyethersulfon)-Filter verwendet werden,

Ein solches Filtergewebe auslassseitig vorzusehen, verhindert, dass Partikel aus der Tropfkammer in den Auslassanschluss und somit zum Auslassschlauch und Patientenanschluss gelangen.

Durch den Einsatz eines hydrophilen PES kann durch deren spezielle hydrophile Eigenschaft ein Leerlaufschutz der Tropfkammer realisiert werden.

Mit Vorteil ist gemäß Anspruch 10 das obere Ende der Tropfkammer aus ABS (Acrylnitril-Butadien-Styrol-Copolymerisat) und der untere Teil der Tropfkammer, also der Teil der unterhalb des oberen Endes angeordnet ist, aus PP/SEB (Polypropylen/Styrol-Ethylen-Butylen-Styrol-Copolymer) gebildet.

Eine solche Materialwahl hat sich als besonders günstig und im praktischen Einsatz vorteilhaft erwiesen.

Mit Vorteil wird das Verschlusselement gemäß Anschluss 11 aus Silikon gebildet. Die Wahl von Silikon ermöglicht eine besonders einfache Herstellung und eine zuverlässige Rückstellkraft bei nicht zu großem Widerstand bei der Bedienung bzw. der Verformung des Verschlusselementes.

Mit Vorteil sind gemäß Anspruch 12 der Ballon aus PE (Polyethylen) und die Muffe aus ABS gebildet.

Weitere Vorteile und Mögliche Ausführungsformen sollen beispielhaft an den rein schematischen Figuren beschrieben werden.

Dabei zeigen die Figuren im Einzelnen:
Figur 1 eine Ansicht eines Infusionsbestecks mit Haupt- und Nebenlinie;
Figur 2 einen Zusammenbau einer Tropfkammer;
Figur 3 ein Lüftungsventil;
Figur 4 ein oberes Ende einer Tropfkammer mit einem Lüftungsventil;
Figur 5 eine Ansicht von oben auf ein oberes Ende einer Tropfkammer mit einem Lüftungsventil im geöffneten Zustand;
Figur 6 zwei Querschnitte durch eine Tropfkammer mit Ballon; und
Figur 7 ein Querschnitt durch ein Oberteil einer Tropfkammer.

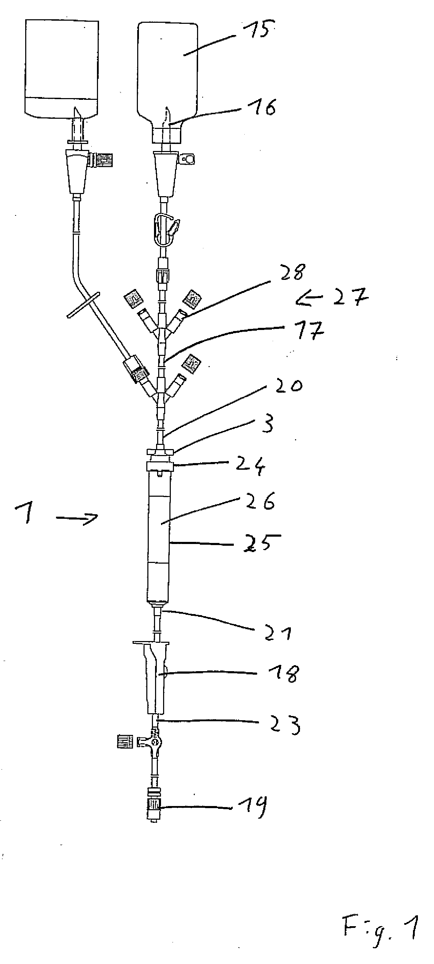

Figur 1 zeigt eine Ansicht eines Infusionsbestecks 27 mit einer Hauptlinie und einer Nebenlinie. In zwei oben angeordnete Infusionsbehältnisse 15 sind jeweils ein Einstechdorn 16 eingebracht. Das rechte Infusionsbehältnis 15 und der rechte Einstechdorn 16 in Figur 1 sind der Hauptlinie zuzuordnen. Nach unten stromabwärts folgt sodann ein Hauptlinienschlauch 17 sowie verschiedene Konnektoren 28. Einem solchen Konnektor 28 ist ein Nebenlinienschlauch zugeführt. Der Nebenlinienschlauch stammt dabei aus dem linken Infusionsbehältnis und dem linken Einstechdorn 16. Nach dem Zusammenfluss von Haupt- und Nebenlinie mündet das Infusionsbesteck 27 in einen Zuflussschlauchanschluss einer Tropfkammer 1. Dieser ist an einem Oberteil 24 der Tropfkammer 1 angeordnet. Dort befindet sich ebenfalls ein Lüftungsventil 3. Unterhalb des Oberteils 24 der Tropfkammer 1 befindet sich ein Unterteil 25 der Tropfkammer 1. Das Unterteil 25 der Tropfkammer 1 und ein Oberteil 24 der Tropfkammer 1 umschließen ein Tropfraumvolumen 26. Im unteren Bereich des Unterteils 25 ist ein Auslassschlauchanschluss 21 angeordnet. Abwärts folgen dann am Auslassschlauch 23 angeordnet ein Durchflussregler 18 und ein Patientenanschluss 19.

Die Infusion kann nun aus dem Infusionsbehältnis über die Schläuche in die Tropfkammer 1 gelangen. Dort kann sichergestellt werden, dass keine Luft in den Auslassschlauch 23 und somit in den Patienten gerät.

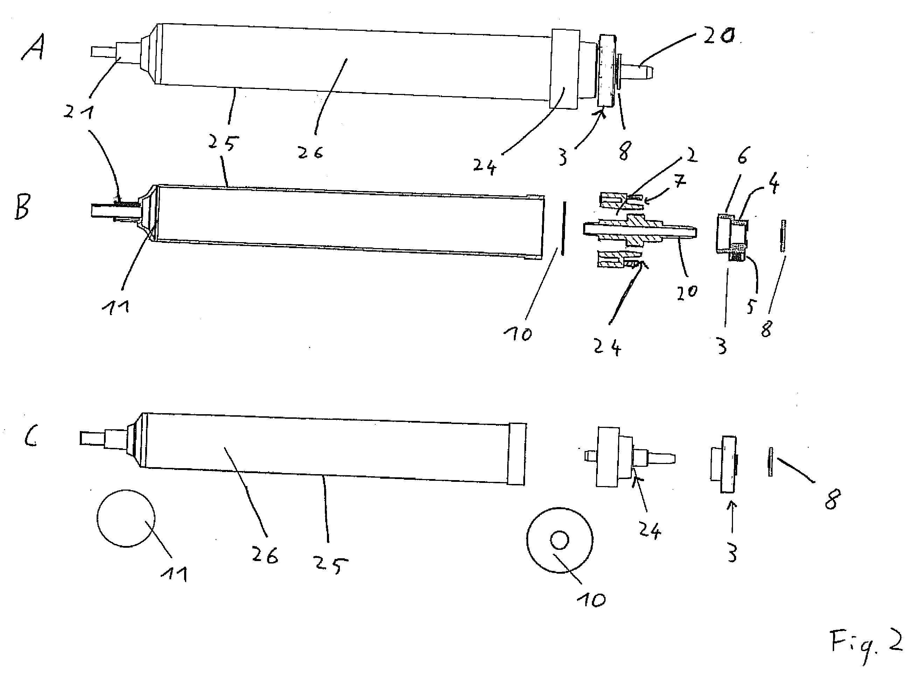

Figur 2 zeigt in seinen Figuren a, b und c den Zusammenbau bzw. den Aufbau von Teilen einer erfindungsgemäßen Tropfkammer 1. Figur 2a zeigt dabei eine zusammengebaute erfindungsgemäße Tropfkammer 1 aufweisend einen Auslassschlauch 21, der am unteren Bereich eines Unterteils 25 der Tropfkammer 1 angeordnet ist. Eingeschlossen vom Unterteil der Tropfkammer 25 und eines Oberteils 24 der Tropfkammer 1 ist ein Tropfkammervolumen 26. Am Oberteil 24 der Tropfkammer 1 ist ein Lüftungsventil 3, gesichert durch ein Sicherungselement 8, angeordnet. Des Weiteren befindet sich dort ein Zuführschlauchanschluss 20.

Figur 2c zeigt die einzelnen Bestandteile in einem demontierten Zustand. Zu erkennen ist das Unterteil 25 der Tropfkammer sowie ein unten in der Tropfkammer einzubringender 15 hydrophiler Auslassfilter 11. Darüber hinaus ist ein 0,2µ hydrophober Filter 10 und das Oberteil 24 der Tropfkammer 1 gezeigt. Ebenfalls separat dargestellt sind das Lüftungsventil 3 und die Sicherungsscheibe 8.

Figur 2b zeigt einen Schnitt durch eine weitgehend auseinander gebaute Tropfkammer 1. Zu erkennen ist im linken Bereich das Unterteil 25 der Tropfkammer 1 in dem vor dem Auslassschlauchanschluss 21 der Auslassfilter 11 angeordnet ist. Am Oberteil 24 der Tropfkammer 1 sind zwei Durchlassöffnungen 2 zu erkennen. Diese Durchlassöffnungen weisen einen gemeinsamen vergrößerten Sammelraum auf, der an den Filter 10 angrenzt, um die Filteroberfläche möglichst effektiv zu nutzen. Im weiteren Verlauf sind die Durchlassöffnungen 2 verjüngt. Des Weiteren zu erkennen sind Vertiefungen 7, in die ein Befestigungsabschnitt 6 des Lüftungsventils 3 eingebracht werden kann. Des Weiteren weist das Oberteil 24 der Tropfkammer mündend in den Zuführschlauchanschluss 20 eine Durchführung für die Infusion auf. Diese erstreckt sich durch das im Filter 10 vorhandene Loch hindurch, sodass die Infusion, die über einen Zuführschlauch und den Zuführschlauchanschluss in die Tropfkammer geführt wird, nicht den Filter 10 passieren muss, sondern durch das in ihm vorhandene Loch hindurchfließen kann.

Das Lüftungsventil weist einen Befestigungsabschnitt 6 auf, der formschlüssig in die am Oberteil 24 vorgesehene ringförmige Vertiefung 7 eingebracht werden kann. Darüber kann das Lüftungsventil 3 am Oberteil 24 befestigt werden. Darüber hinaus weist das Lüftungsventil 3 ein elastisches Verschlusselement 4 auf, das im Ruhezustand die Durchlassöffnungen 2 blockiert. Darüber hinaus ist am Lüftungsventil 3 ein Bedienflügel 5 zu erkennen. Daneben dargestellt ist das Sicherungselement 8, das als Scheibe ausgebildet ist und zur weiteren Befestigung des Lüftungsventils 3 dient.

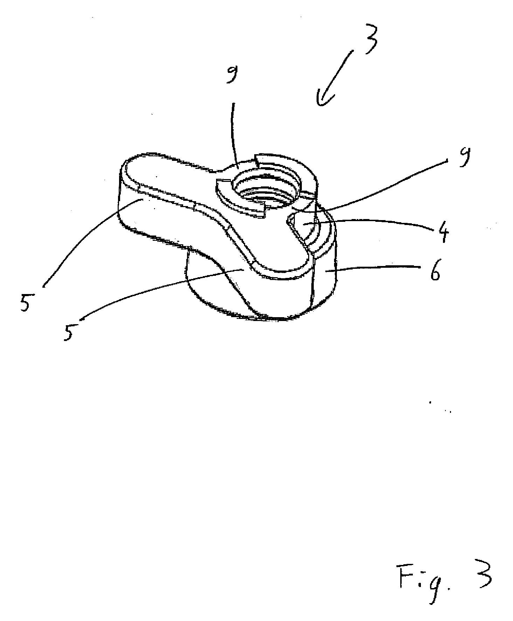

Figur 3 zeigt eine Detailansicht des Lüftungsventils 3. Zu erkennen sind zunächst zwei Flügel 5. Diese sind als radiale Fortsätze an einem ringförmigen Verschlusselement 4 angeordnet. Das ringförmige Verschlusselement 4 weist zwei Gasauslassaussparungen 9 auf. Darüber hinaus weist das Lüftungsventil einen ringförmigen Befestigungsabschnitt 6 auf. Im hier gezeigten Ruhezustand des Lüftungsventils 3 ist das elastische Verschlusselement 4 ringförmig. Werden die beiden Bedienflügel 5 zusammengedrückt, also aufeinander zu bewegt, wird dadurch das elastische Verschlusselement 4 verformt. Dadurch werden die Durchlassöffnungen 2 freigegeben.

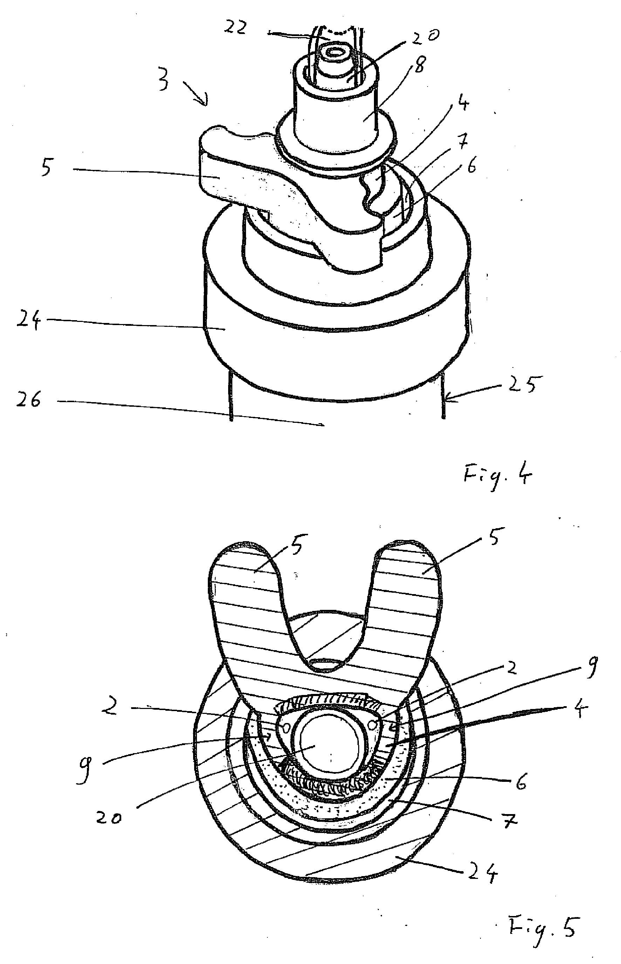

Figur 4 zeigt einen Einbau eines Lüftungsventils 3 aus Figur 3 in einer erfindungsgemäßen Tropfkammer 1. Zu erkennen ist ein kleiner Abschnitt des Unterteils 25, an den ein Oberteil 24 angrenzt. Das obere Element 24 weist eine ringförmige Vertiefung 7 auf. In diese Vertiefung 7 ist der ringförmige Befestigungsabschnitt 6 des Lüftungsventils 3 eingebracht. Das Lüftungsventil 3 ist dabei konzentrisch um einen Zuführschlauchanschluss 20 angeordnet. Zu erkennen ist auch ein Teil des ringförmigen Verschlusselementes 4 und die zwei Bedienflügel 5. Das Lüftungsventil 3 ist darüber hinaus über ein ebenfalls konzentrisch zum Zuführschlauchanschluss 20 angeordnetes Sicherungselement 8 gesichert. Dieses Sicherungselement 8 klemmt darüber hinaus einen Zuführschlauch 22 auf den Zuführschlauchanschlussanschluss 20.

Figur 5 zeigt die Anordnung aus Figur 4, jedoch von oben und ohne Sicherungselement 8 und Zuführschlauch 22. Darüber hinaus befindet sich das Lüftungsventil 3 in Figur 5 nicht im Ruhezustand, sondern im geöffneten Zustand. Dazu sind die Bedienflügel 5 aufeinander zu bewegt. Dieses hat zu einer Verformung des Verschlusselementes 4 geführt. Dadurch hat das Verschlusselement 4 die Durchlassöffnungen 2 freigegeben. Durch diese kann nun Gas in das oder aus dem Tropfraumvolumen strömen. Damit das Gas auch bei aufgesetztem Sicherungselement 8 ungehindert strömen kann, sind die Gasauslassaussparungen vorgesehen. Diese sind gegenüber den Darstellungen in Figur 3 ebenfalls durch die Betätigung der Bedienflügel 5 verzerrt. Zu erkennen ist in Figur 5 auch der ringförmige Befestigungsabschnitt 6, der ebenfalls leicht verformt ist. Durch eine solche Verformung bei Betätigung der Bedienflügel 5 kann der ringförmige Befestigungsabschnitt 6 ebenfalls zur Rückstellkraft, die ansonsten vor allem durch das ringförmige Verschlusselement ausgeübt wird, verstärkt werden. Durch die Verformung des ringförmigen Befestigungselementes ist auch ein Teil der Vertiefung 7 erkennbar.

Werden die Bedienflügel 5 losgelassen, so kehrt das ringförmige Verschlusselement 4 selbsttätig wieder in seine ursprüngliche ringförmige und konzentrisch um den Durchführschlauchanschluss 20 angeordnete Form zurück und verschließt dadurch die Durchlassöffnungen 2. Dabei kehrt auch das ringförmige Befestigungselement in seine ursprüngliche ringförmige und ebenfalls konzentrisch um den Zuführschlauchanschluss 20 angeordnete Form zurück.

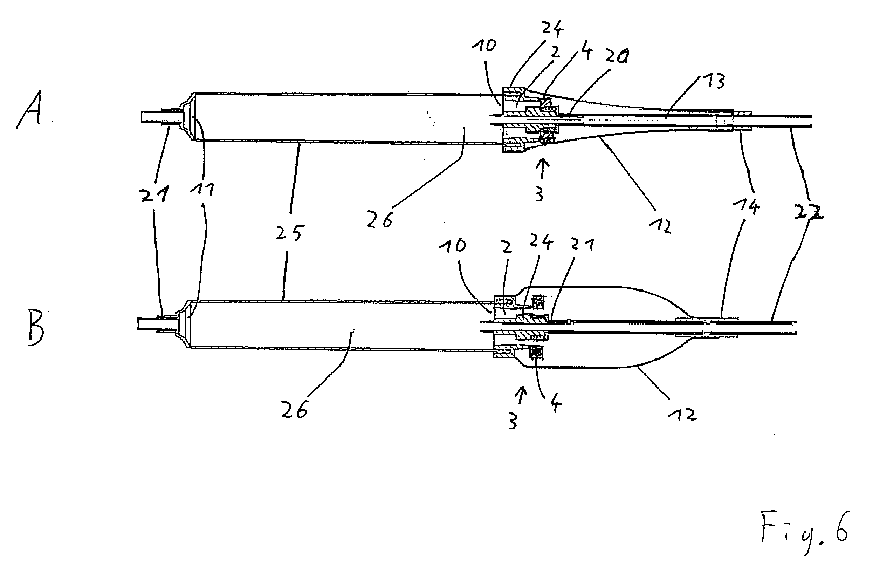

Figur 6 zeigt zwei Querschnittsansichten einer erfindungsgemäßen Tropfkammer mit Ballon. Dabei zeigt Figur 6a solche im Auslieferungszustand mit weitgehend geleertem Ballon und Figur 6b eine solche mit gefülltem Ballon.

In Figur 6a ist zu erkennen, dass an dem Oberteil 24 der Tropfkammer 1 im äußeren Umfang ein Ballon 12 angesetzt ist. Des Weiteren befindet sich am Zuführschlauchanschluss ein befestigtes Schlauch 13, an dem wiederum eine Muffe 14 befestigt ist. Diese Muffe 14 ist so ausgestaltet, dass an ihr ein Zuführschlauch 22 befestigt werden kann. Des Weiteren ist an der Muffe 14 der Ballon 12 befestigt. Zu erkennen ist des Weiteren ein Lüftungsventil im Ruhezustand, das zwei Durchlassöffnungen 2 im Oberteil 24 der Tropfkammer 1 verschießt. Ebenfalls gezeigt sind ein Filter 10 sowie ein Auslassfilter und ein Auslassschlauchanschluss 21. Eingeschlossen von einem Unterteil 25 und einem Oberteil 24 befindet sich ein Tropfkammervolumen 26.

Figur 6b zeigt die Anordnung aus Figur 6a jedoch mit geöffnetem Lüftungsventil 3 und weitestgehend gefülltem Ballon 12. Zu erkennen ist, dass das Lüftungsventil im geöffneten Zustand gezeigt ist, sodass das ringförmige Verschlusselement 4 die zwei Durchlassöffnungen 2 nicht blockiert und Gas zwischen Ballon und Tropfkammervolumen strömen kann.

Dabei ist zu erkennen, dass jegliches Gas, das durch die 0,2µ hydrophobe Filtermembran filtriert und durch die Durchlassöffnungen 2 strömt, in dem Ballon 12 aufgefangen wird. Dies ist dadurch gewährleistet, dass der Ballon 12 nicht am Lüftungsventil 3 befestigt ist, sondern direkt am Oberteil 24 angebracht ist.

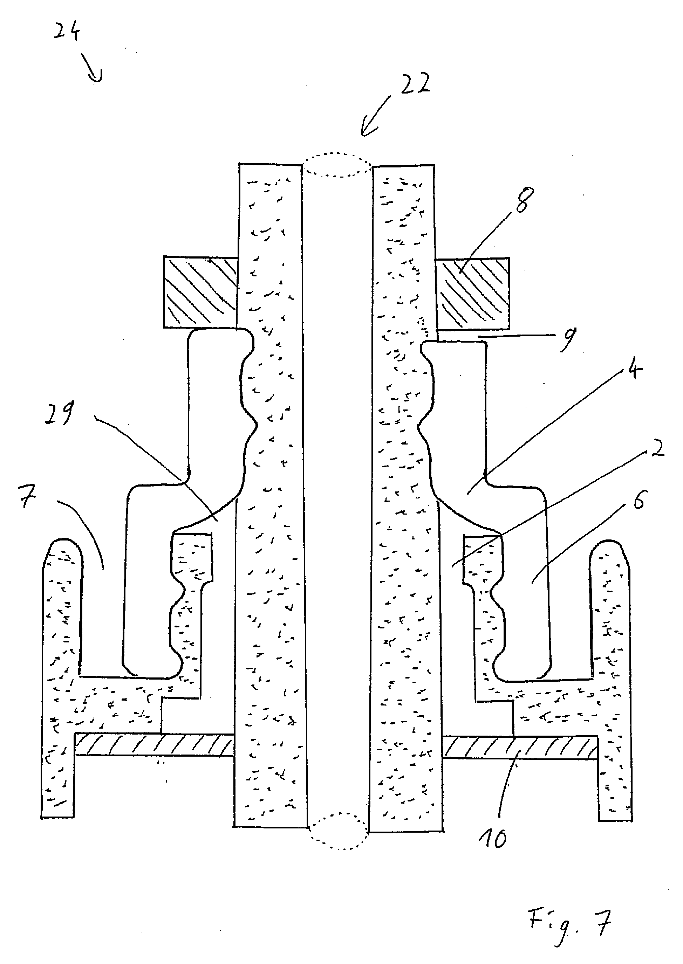

Fig. 7 zeigt einen Querschnitt durch ein Oberteil 24. Zu erkennen ist ein Sicherungselement 8, ein Zuführanschluss 22, ein Verschlusselement 4 mit Befestigungsabschnitt 6, sowie ein hydrophober Filter 10, Durchlassöffnungen 2, eine ringförmige Vertiefung 7, eine Gasauslassaussparung 9 und ein Lüftungskanal 29.

Das Verschlusselement 4 dichtet im Ruhezustand mit einer kleinen Unterbrechung in Höhe des umlaufenden Lüftungskanals 29. Dabei dient sein unterer Abschnitt gleichzeitig als Befestigungsabschnitt 6. Das Tropfkammervolumen steht über den hydrophoben Filter 10 in Verbindung mit den Durchlassöffnungen 2, die in den umlaufenden Lüftungskanal 29 münden. Dieser ist zur Umgebung im Ruhezustand durch das wie oben beschrieben abdichtende Verschlusselement 29 abgedichtet.

Das Verschlusselement 4, das durch das Sicherungselement 8 gesichert ist, weist an seinem oberen Ende über einige Winkelbereicht Gasauslassaussparungen 9 auf.

Wird das Verschlusselement zur Öffnung des Lüftungsventils elastisch verformt, wird der umlaufende Lüftungskanal 29 mit mindestens einer Gasauslassaussparung 9 in Verbindung gebracht, so dass Gas aus dem inneren der Tropfkammer über den hydrophoben Filter 10 und die Durchlassöffnungen 2 und den Lüftungskanal 29 sowie den durch das elastische Verformen geschaffenen Raum und die Gasauslassaussparungen 9 mit der Umgebung ausgetauscht werden kann. So ist ein Be- und Entlüften der Tropfkammer möglich.

Weitere Ausbildungen, die auf den jeweiligen Anwendungsfall angepasst sein können, sind durch den Fachmann leicht auffindbar.

**Bezugszeichenliste**

| | | | |
|---|---|---|---|
| 1 | Tropfkammer | 16 | Einstechdorn |
| 2 | Durchlassöffnung | 17 | Hauptlinienschlauch |
| 3 | Lüftungsventil | 18 | Durchflussregler |
| 4 | Elastisches Verschlusselement | 19 | Patientenanschluss |
| | | 20 | Zuführschlauchanschluss |
| 5 | Bedienflügel | 21 | Auslassschlauchanschluss |
| 6 | Befestigungsabschnitt | 22 | Zuführschlauch |
| 7 | Vertiefung | 23 | Auslassschlauch |
| 8 | Sicherungselement | 24 | Oberteil |
| 9 | Gasauslassaussparung | 25 | Unterteil |
| 10 | Hydrophobe Filtermembran | 26 | Tropfkammervolumen |
| 11 | Hydrophiler Auslassfilter | 27 | Infusionsbesteck |
| 12 | Flexibler Ballon | 28 | Konnektor |
| 13 | Schlauch | 29 | Umlaufender Lüftungskanal |
| 14 | Muffe | | |
| 15 | Infusionsbehältnis | | |

## Patentansprüche

1. Tropfkammer (1) für ein Infusionsbesteck (27) zur Abgabe einer Infusions- und/oder Medikamenten-Flüssigkeit an einen Patienten, aufweisend einen an einem oberen Ende (24) der Tropfkammer angeordneten Zuführschlauchanschluss (20) und einen an einem unteren Ende der Tropfkammer angeordneten Auslassschlauchanschluss (21), wobei am oberen Ende (24) der Tropfkammer (1) mindestens eine Durchlassöffnung (2) ausgebildet ist und dass ein Lüftungsventil (3) an dem oberen Ende (24) der Tropfkammer (1) zur Be- und /oder Entgasung derselben vorgesehen ist, und dass das Lüftungsventil (3) ein elastisches Verschlusselement (4) zum automatischen Verschließen der mindestens einen Durchlassöffnung (2) und zwei Bedienflügel (5) zum manuellen elastischen Verformen des Verschlusselementes (4) unter zumindest teilweiser Freigabe der mindestens einen Durchlassöffnung (2) umfasst, **dadurch gekennzeichnet, dass** an dem oberen Ende (24) der Tropfkammer (1) ein flexibler Ballon (12) zur Aufnahme des durch die mindestens eine Durchlassöffnung (2) austretenden Entlüftungsgas angeordnet ist und dass an dem Zuführschlauchanschluss (20) ein Rohr oder Schlauch (13) mit einer Muffe (14) zum Anschluss eines Zuführschlauches (22) angeordnet ist und der Ballon (12) an der Muffe (14) fixiert ist.

2. Tropfkammer (1) nach Anspruch 1, **dadurch gekennzeichnet, dass** das Lüftungsventil (3) so ausgebildet ist, dass die Bedienflügel (5) mit zwei Fingern unter der manuellen elastischen Verformung zur zumindest teilweisen Freigabe der mindestens einen Durchlassöffnung (2) aufeinander zu bewegbar sind.

3. Tropfkammer (1) nach einem der vorstehenden Ansprüche, **dadurch gekennzeichnet, dass** mindestens zwei, insbesondere vier, Durchlassöffnungen (2) vorgesehen sind.

4. Tropfkammer (1) nach Anspruch 3, **dadurch gekennzeichnet, dass** das Verschlusselement (4) ringförmig ausgebildet und konzentrisch um den Zuführschlauchanschluss (20) angeordnet ist.

5. Tropfkammer (1) nach Anspruch 5, **dadurch gekennzeichnet, dass** sich die Bedienflügel (5) vom Verschlusselement (4) aus radial nach außen erstrecken und insbesondere einen Winkel von mindestens 40° und weniger als 180° einschließen.

6. Tropfkammer (1) nach einem der vorstehenden Ansprüche, **dadurch gekennzeichnet, dass** das Lüftungsventil (3) einen ringförmigen Befestigungsabschnitt (6) zum formschlüssigen Eingriff in eine am oberen Ende (24) der Tropfkammer (1) vorgesehene ringförmige und zum Zuführschlauchanschluss (20) konzentrische Vertiefung (7) aufweist.

7. Tropfkammer (1) nach einem der vorstehenden Ansprüche, **dadurch gekennzeichnet, dass** ein Sicherungselement (8) zur Sicherung des Lüftungsventils (3) oberhalb und direkt angrenzend an dass das Verschlusselement (4) vorgesehen ist und das Verschlusselement an einem oberen an das Sicherungselement (8) angrenzenden Ende mindestens eine Gasauslassaussparung (9) aufweist.

8. Tropfkammer (1) nach einem der vorstehenden Ansprüche, **dadurch gekennzeichnet, dass** eine Filtermembran (10), insbesondere eine 0,2µ hydrophobe Filtermembran (10) mit einer Filtrationsfläche von mindestens 85mm², zwischen der mindestens einen Durchlassöffnung (2) und dem Tropfkammervolumen (26) integriert ist.

9. Tropfkammer (1) nach einem der vorstehenden Ansprüche, **dadurch gekennzeichnet, dass** zwischen Tropfkammervolumen (26) und Auslassanschluss (21) ein, insbesondere aus PA (Polyamid)- oder hydrophilem PES (Polyethersulfon)-Filtergewebe gebildeter, Auslassfilter (11) vorgesehen ist.

10. Tropfkammer (1) nach einem der vorstehenden Ansprüche, **dadurch gekennzeichnet, dass** das obere Ende (24) aus ABS (Acrylnitril-Butadien-Styrol-Copolymerisat) und der unterhalb des oberen Endes (24) angeordnete Teil (25) aus PP/SEB (Polypropylen/Styrol-Ethylen-Butylen-Styrol-Copolymer) bestehen.

11. Tropfkammer (1) nach einem der vorstehenden Ansprüche, **dadurch gekennzeichnet, dass** das elastische Verschlusselement (4) aus Silikon besteht.

12. Tropfkammer (1) nach Anspruch 1, **dadurch gekennzeichnet, dass** der Ballon (12) aus PE (Polyethylen) und die Muffe (14) aus ABS bestehen.

## Claims

1. Drip chamber (1) for an infusion set (27) for administering an infusion and/or medication liquid to a patient, having an inlet tubing connection (20) located at the upper end (24) of the drip chamber and an outlet tubing connection (21) at the lower end of the drip chamber, wherein at least one flow passage (2) is formed at the upper end (24) of the drip chamber (1) and that a ventilation valve (3) at the upper end (24) of the drip chamber (1) is provided for the gassing and /or venting of the same, and that the ventilation valve (3) includes an elastic closure element (4) for the automatic closing of the at least one flow passage (2) and two operating lobes (5) for manual elastic deformation of the closure element (4) to enable at least partial release of the at least one flow passage (2), **characterised in that** a flexible balloon (12) for accommodating the venting gas exiting through a flow passage (2) is located at the upper end (24) of the drip chamber (1) and that a tube or tubing (13) with a sleeve (14) for connecting infeed tubing (22) at the inlet feed tubing connection (20) and the balloon (12) is fixed to the sleeve (14).

2. Drip chamber (1) according to claim 1, **characterised in that** the ventilation valve (3) is so formed that the operating lobes (5) can be pressed together with two fingers to perform manual elastic deformation enabling an at least partial release of the at least one flow passage (2).

3. Drip chamber (1) according to one of the foregoing claims, **characterised in that** at least two, especially four, flow passages are provided.

4. Drip chamber (1) according to claim 3, **characterised in that** the closing element (4) is ring-shaped and located concentrically about the inlet tubing connection (20).

5. Drip chamber (1) according to claim 5, **characterised in that** the operating lobes (5) of the closing element (4) point radially outwards and especially include an angle of at least 40° and less than 180°.

6. Drip chamber (1) according to one of the foregoing claims, **characterised in that** the ventilation valve (3) has a ring-shaped fastening section (6) for positive mechanical engagement in a ring-shaped concentric recess (7) provided in the infeed tubing connection (20) at the upper end (24) of the drip chamber (1).

7. Drip chamber (1) according to one of the foregoing claims, **characterised in that** a securing element (8) for the securing of the ventilation valve (3) above and directly adjacent to the closure element (4) is provided and that the closure element (4) has at least one gas outlet cut-out (9) at an upper end adjacent to the securing element (8).

8. Drip chamber (1) according to one of the foregoing claims, **characterised in that** a filter membrane (10), especially a 0.2µ hydrophobic filter membrane (10) with a filtration area of at least 85mm², is incorporated between the at least one flow passage (2) and the drip chamber space (26).

9. Drip chamber (1) according to one of the foregoing claims, **characterised in that** an outlet filter (11), especially of PA (polyamide) or hydrophilic PES (polyethersulfone) filter fabric, is provided between the drip chamber space (26) and outlet connection (21).

10. Drip chamber (1) according to one of the foregoing claims, **characterised in that** the upper end (24) comprises ABS (acrylonitrile butadiene styrene copolymer) and the part (25) located underneath the upper end (24) comprises PP/SEB (polypropylene/styrene ethylene butylene styrene copolymer).

11. Drip chamber (1) according to one of the foregoing claims, **characterised in that** the elastic closure element (4) comprises silicone.

12. Drip chamber (1) according to claim 1, **characterised in that** the balloon (12) comprises PE (polyethylene) and the sleeve (14) comprises ABS.

## Revendications

1. Chambre compte-gouttes (1) pour un dispositif de perfusion (27) permettant l'administration à un patient d'un liquide de perfusion et/ou médicamenteux, présentant un raccord de tuyau d'amenée (20) disposé à une extrémité supérieure (24) de la chambre compte-gouttes et un raccord de tuyau de sortie (21) disposé à une extrémité inférieure de la chambre compte-gouttes, où au moins une ouverture d'écoulement (2) est ménagée à l'extrémité supérieure (24) de la chambre compte-gouttes (1) et une valve d'aération (3) est prévue à l'extrémité supérieure (24) de la chambre compte-gouttes (1) pour l'aération et/ou la purge de celle-ci, où la valve d'aération comprend un élément de fermeture élastique (4) pour la fermeture automatique de ladite au moins une ouverture d'écoulement (2) et deux ailettes de commande (5) pour la déformation élastique manuelle de l'élément de fermeture (4) avec une libération au moins partielle de ladite au moins une ouverture d'écoulement (2), **caractérisée en ce qu'**à l'extrémité supérieure (24) de la chambre compte-gouttes (1) est disposé un ballonnet flexible (12) destiné à recueillir le gaz de purge sortant de ladite au moins une ouverture d'écoulement (2), et **en ce qu'**un conduit ou un tuyau (13) avec un manchon (14) pour la connexion d'un tuyau d'amenée (22) est disposé contre le raccord de tuyau d'amenée (20), et le ballonnet (12) est fixé contre le manchon (14).

2. Chambre compte-gouttes (1) selon la revendication 1, **caractérisée en ce que** la valve d'aération (3) est réalisée de manière à permettre un déplacement l'une vers l'autre des ailettes de commande (5) au moyen de deux doigts avec déformation élastique manuelle de l'élément de fermeture (4) pour une libération au moins partielle de ladite au moins une ouverture d'écoulement (2).

3. Chambre compte-gouttes (1) selon l'une des revendications précédentes, **caractérisée en ce qu'**au moins deux, en particulier quatre ouvertures d'écoulement sont prévues.

4. Chambre compte-gouttes (1) selon la revendication 3, **caractérisée en ce que** l'élément de fermeture (4) est réalisé en forme d'anneau et est disposé de manière concentrique autour du raccord de tuyau d'amenée (20).

5. Chambre compte-gouttes (1) selon la revendication 5, **caractérisée en ce que** les ailettes de commande (5) s'étendent radialement vers l'extérieur depuis l'élément de fermeture (4) et forment en particulier un angle égal ou supérieur à 40° et inférieur à 180°.

6. Chambre compte-gouttes (1) selon l'une des revendications précédentes, **caractérisée en ce que** la valve d'aération (3) comporte une partie de fixation annulaire (6) pour un engagement par correspondance de forme dans un évidement (7) annulaire prévu à l'extrémité supérieure (24) de la chambre compte-gouttes (1) et concentrique au raccord de tuyau d'amenée (20).

7. Chambre compte-gouttes (1) selon l'une des revendications précédentes, **caractérisée en ce qu'**un élément de fixation (8) pour la fixation de la valve d'aération (3) est prévu au-dessus de l'élément de fermeture (4) et directement adjacent à celui-ci, et **en ce que** l'élément de fermeture présente un évidement d'échappement de gaz (9) à une extrémité supérieure adjacente à l'élément de fixation (8).

8. Chambre compte-gouttes (1) selon l'une des revendications précédentes, **caractérisée en ce qu'**une membrane de filtre (10), en particulier une membrane de filtre (10) hydrophobe de 0,2 µ ayant une surface de filtration égale ou supérieure à 85mm², est intégrée entre ladite au moins une ouverture d'écoulement (2) et le volume (26) de la chambre compte-gouttes.

9. Chambre compte-gouttes (1) selon l'une des revendications précédentes, **caractérisée en ce qu'**un filtre de sortie (11), en particulier constitué d'un tissu filtre en PA (polyamide) ou en PES (polyéthersulfone) hydrophile est prévu entre le volume (26) de la chambre compte-gouttes et le raccord de sortie (21).

10. Chambre compte-gouttes (1) selon l'une des revendications précédentes, **caractérisée en ce que** l'extrémité supérieure (24) est en ABS (copolymère d'acrylonitrile-butadiène-styrène) et la pièce (25) disposée n dessous de l'extrémité supérieure (24) en PP/SEB (copolymère de polypropylène/styrène-éthylène-butylène-styrène).

11. Chambre compte-gouttes (1) selon l'une des revendications précédentes, **caractérisée en ce que** l'élément de fermeture (4) élastique est en silicone.

12. Chambre compte-gouttes (1) selon la revendication 1, **caractérisée en ce que** le ballonnet (12) est en PE (polyéthylène) et le manchon (14) en ABS.
